(19) Europäisches Patentamt
European Patent Office
Office européen des brevets

(11) Veröffentlichungsnummer: **0 275 024 B1**

# (12) EUROPÄISCHE PATENTSCHRIFT

(45) Veröffentlichungstag der Patentschrift: **04.09.91**

(51) Int. Cl.5: **C07D 333/24**, C07D 333/16, C07D 333/18, A61K 31/38

(21) Anmeldenummer: **88100087.1**

(22) Anmeldetag: **07.01.88**

Die Akte enthält technische Angaben, die nach dem Eingang der Anmeldung eingereicht wurden und die nicht in dieser Patentschrift enthalten sind.

## (54) Neue Carbonsäurederivate, Verfahren zu ihrer Herstellung sowie Arzneimittel, die diese Verbindungen enthalten.

(30) Priorität: **13.01.87 DE 3700732**

(43) Veröffentlichungstag der Anmeldung:
**20.07.88 Patentblatt 88/29**

(45) Bekanntmachung des Hinweises auf die Patenterteilung:
**04.09.91 Patentblatt 91/36**

(84) Benannte Vertragsstaaten:
**AT BE CH DE ES FR GB GR IT LI LU NL SE**

(56) Entgegenhaltungen:
**DE-A- 2 810 262**
**FR-A- 2 400 507**

(73) Patentinhaber: **BOEHRINGER MANNHEIM GMBH**
**Sandhofer Strasse 116**
**W-6800 Mannheim 31(DE)**

(72) Erfinder: **Wolff, Hans Peter, Dr. rer. nat.**
**Rebenweg 24**
**W-6945 Hirschberg-Grosssachsen(DE)**
Erfinder: **Kühnle, Hans-Frieder, Dr. rer. nat.**
**Silcher Weg 6**
**W-6940 Weinheim(DE)**

EP 0 275 024 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung sind neue Carbonsäurederiv der allgemeinen Formel I

$$\begin{array}{l} R_1\text{-A-CH-COOH} \\ \qquad\quad | \\ \qquad\ \text{Y-B-}R_2 \end{array} \qquad (I)$$

in welcher

- $R_1$ und $R_2$ unabhängig voneinander einen gegebenenfalls durch Fluor, Chlor, Brom, $C_1$-$C_6$ Alkyl, $C_1$-$C_6$ Alkoxy, Trifluormethyl oder Cyano substituierten $C_6$-$C_{14}$ Arylrest oder einen gegebenenfalls durch Fluor, Chlor, Brom $C_1$-$C_6$ Alkyl oder $C_1$-$C_6$ Alkoxy substituierten Thiophen-, Pyridin- oder Furanrest,
- A einen geradkettigen oder verzweigten, gesättigten oder ungesättigten zweiwertigen aliphatischen Kohlenwasserstoffrest mit 1 bis 10 Kohlenstoffatomen, der ein Sauerstoff-, Schwefel- oder Stickstoffatom als Kettenglied enthalten kann und gegebenenfalls durch einen Hydroxylrest substituiert ist,
- Y die Gruppe $S(O)_n$ oder O,
- n die Zahlen 0, 1 oder 2 und
- B einen Valenzstrich oder einen gesättigten oder ungesättigten zweiwertigen aliphatischen Kohlenwasserstoffrest mit 1 bis 5 Kohlenstoffatomen

bedeuten,
sowie deren physiologisch unbedenkliche Salze, Ester und Amide, mit der Maßgabe, daß

a) $R_1$ und $R_2$ nicht gleichzeitig einen Arylrest bedeuten dürfen und

b) im Falle eines ungesättigten Kohlenwasserstoffrestes A, der die ein Heteroatom enthält, das Heteroatom nicht an einem ungesättigten aliphatischen Kohlenstoffatom gebunden sein darf,

Verfahren zu ihrer Herstellung sowie Arzneimittel, die diese Verbindungen enthalten.

Die Verbindungen der allgemeinen Formel I besitzen wertvolle pharmakologische Eigenschaften. Sie sind als Arzneimittel zur Behandlung von Diabetes, Praediabetes und insbesondere zur Behandlung von Altersdiabetes geeignet.

Die Verbindungen der Formel I haben zu bekannten Antidiabetica strukturell und in der Wirkungsart keine Beziehung. Sie senken den Blutzuckerspiegel durch Verstärkung der peripheren Glucoseoxidation. Ihre Wirkung beruht auf einer Erhöhung der Sensitivität von peripherem Gewebe gegenüber Insulin. Im Gegensatz zu den Biguaniden wird dabei kein Anstieg der Blutlaktatwerte beobachtet. Die Verbindungen der allgemeinen Formel I stellen daher auch eine wertvolle Bereicherung bei der Behandlung nicht-diabetischer Krankheitszustände dar, bei denen eine Insulinresistenz vorhanden ist, wie z. B. Adipositas und Atherosklerose.

Zusätzlich zeigen sie eine ausgeprägte Lipidsenkung und eignen sich daher auch zur Behandlung von Fettstoffwechselerkrankungen.

Als Arylreste mit 6 bis 14 Kohlenstoffatomen sind in allen Fällen aromatische Kohlenwasserstoffe, insbesondere die Phenylgruppe, zu verstehen. Diese Arylreste können in allen möglichen Positionen ein- oder mehrfach wie angegeben substituiert sein.

Bevorzugt in diesem Sinne ist beispielsweise die Methyl-, tert.-Butyl- oder Methoxygruppe.

Der Thiophen-, der Pyridin-und der Furanrest können in allen möglichen Positionen ein- oder mehrfach wie angegeben substituiert sein.

Bevorzugte Substituenten sind Chlor, Methyl, Ethyl und Methoxy.

Für unverzweigte Kohlenwasserstoffreste A kommen bevorzugt die folgenden in Betracht:

$-CH=CHCH_2-$, $-C\equiv CCH_2-$,
$-(CH_2)_m-$, $-(CH_2)_r-X-(CH_2)_s-$,
$-CH=CHCH_2-X-(CH_2)_t-$ und $-C\equiv CCH_2-X-(CH_2)_t-$,

in welchen

- m eine ganze Zahl von 1 bis 10
- r und

s zwei ganze Zahlen von 0 bis 10, deren Summe gleich m ist,
t eine ganze Zahl von 1 bis 7 und
X Sauerstoff, Schwefel oder Stickstoff

bedeutet.

Als verzweigte Kohlenwasserstoffreste A sind insbesondere bevorzugt

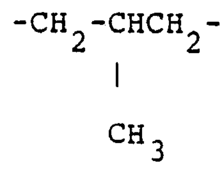 und 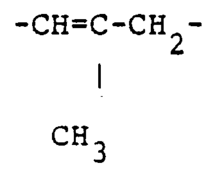

B bedeutet eine Bindung oder einen 1 bis 5 Kohlenstoffatome enthaltenden zweiwertigen Kohlenwasserstoffrest. Bevorzugt sind die folgenden Alkylenketten:
$-CH_2-$, $-(CH_2)_2-$, $-(CH_2)_3-$, $-CH=CH-$, $-CH=CHCH_2-$ sowie

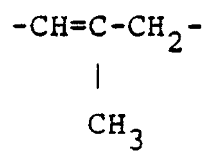

Als physiologisch unbedenkliche Salze kommen insbesondere Alkali-, Erdalkali- oder Ammoniumsalze (sowie gegebenenfalls Salze mit blutzuckersenkenden Biguaniden) in Frage.

Die von den Carbonsäuren der allgemeinen Formel I abgeleiteten Ester enthalten als Alkoholkomponente niedere einwertige Alkohole, von denen Methanol, Ethanol und n-Butanol bevorzugt sind, sowie mehrwertige Alkohole, wie z. B. Glycerin, oder Alkohole mit anderen funktionellen Gruppen, wie z. B. Ethanolamin.

Die erfindungsgemäßen, von den Carbonsäuren der allgemeinen Formel I abgeleiteten Amide enthalten als Aminkomponente vorzugsweise Ammoniak, p-Aminobenzoesäure, β-Alanin, Ethanolamin oder 2-Aminopropranol. Es kommen aber auch Alkylamine, wie z. B. Isopropylamin oder tert.-Butylamin, Dialkylamine, wie Diethylamin, sowie cyclische Amine, wie z. B. Morpholin, oder 4-substituierte Piperazine in Frage.

Die substituierten Carbonsäuren der allgemeinen Formel I besitzen ein Chiralitätszentrum. Die obige Definition der erfindungsgemäßen Verbindungen schließt daher auch alle möglichen Enantiomeren, ihre Gemische und die Racemate ein.

Die Herstellung der Verbindungen der allgemeinen Formel I ist dadurch gekennzeichnet, daß man

A) eine Gruppe der allgemeinen Formel I

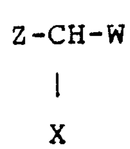 (II),

in welcher

W $-COOR_3$ oder eine andere in die Carboxylfunktion überführbare Gruppe,
$R_3$ eine $C_1$-$C_6$ Alkylgruppe,
X hier und in allen folgenden Erläuterungen eine reaktive Gruppe und
Z Wasserstoff oder die Gruppe $R_1$-A-, in welcher $R_1$ und A die oben angegebene Bedeutung haben,

darstellt,

in an sich bekannter Weise mit einer Gruppe der allgemeinen Formel III

$R_2$-B-YH (III),

in welcher $R_2$, B und Y die oben angegebene Bedeutung haben, zu einer Verbindung der allgemeinen Formel IV

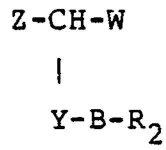
(IV),

umsetzt und im Anschluß daran gegebenenfalls

a) für den Fall, daß Y ein Schwefelatom darstellt, zur Gruppe Y = SO oder $SO_2$ oxidiert oder

b) für den Fall, daß Z Wasserstoff bedeutet, entweder

$b_1$) mit einer Verbindung der allgemeinen Formel V

$R_1$-A-X (V),

alkyliert oder

$b_2$) mit einer Verbindung der allgemeinen Formel V'

$R_1$-A'-CHO (V'),

in welcher A' einen um die $CH_2$-Gruppe verkürzten Kohlenwasserstoffrest A nach oben genannter Definition bedeutet, kondensiert und im Anschluß an die Kondensation die entstandene Doppelbindung hydriert,

oder dass man

B) eine Verbindung der allgemeinen Formel VI

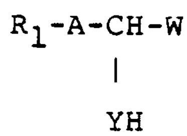
(VI)

mit einer Gruppe der allgemeinen Formel VII

$R_2$-B-X (VII)

zu einer Verbindung der allgemeinen Formel IV umsetzt und an den erhaltenen Verbindungen der allgemeinen Formel IV im Anschluß an die vorgenannten Verfahrensschritte die Gruppe W in eine freie Carboxylfunktion, einen physiologisch verträglichen Ester oder ein Amid umwandelt.

Die Herstellung der Ausgangsverbindungen kann nach an sich bekannten Methoden erfolgen, indem man z. B. Malonester mit Verbindungen der Formel V alkyliert und die erhaltenen Verbindungen der Formel VIII

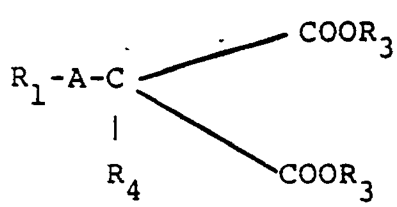
(VIII),

in welchen $R_1$, A und $R_3$ die oben angegebene Bedeutung haben und $R_4$ Wasserstoff ist, nach an sich bekannten Methoden zu Verbindungen VIII umsetzt, in denen $R_4$ eine reaktive Gruppe X darstellt, diese anschließend durch Decarboxylierung in Verbindungen der allgemeinen Formel II überführt, in welcher W

die Gruppe -$COOR_3$ und Z die Gruppe $R_1$-A- bedeutet, und diese gegebenenfalls in Verbindungen der allgemeinen Formel VI umwandelt.

Die Umsetzung der reaktiven Carbonsäurederivate der allgemeinen Formel II mit den Verbindungen der allgemeinen Formel III erfolgt zweckmäßig unter Zusatz eines säurebindenden Mittels, wie z. B. Natriumhydrogencarbonat, Natriumethylat oder Natriumhydrid. Vorzugsweise werden für die Umsetzung Ester der reaktiven Carbonsäurederivate verwendet. Als inertes Lösungsmittel gelangen z. B. Ether, Ethanol, Benzol, Tetrahydrofuran, Dioxan oder Methylenchlorid zum Einsatz. Beim Einsatz anorganischer Basen verwendet man als Reaktionsmedium auch z. B. 2-Butanon, Dimethylformamid, Hexamethylphosphorsäuretriamid oder Acetonitril. Als reaktiver Rest X kommen hier z. B. Halogenide oder Sulfonsäureester in Frage, wie z. B. Chloride, Bromide oder p-Toluolsulfonyl- und Methansulfonyloxygruppen.

Die Oxidation von Verbindungen der allgemeinen Formel IV, in welchen Y S bedeutet, zu Sulfoxiden und Sulfonen wird vorzugsweise mit Wasserstoffperoxid in polaren Lösungsmitteln, wie Eisessig, einem Gemisch von Eisessig und Acetanhydrid oder Aceton durchgeführt. Besonders vorteilhaft hat sich die Oxidation mit Trifluorperessigsäure erwiesen. Als Lösungsmittel wird hierbei zweckmäßig Trifluoressigsäure verwendet.

Die Umsetzung der reaktiven Derivate V mit Verbindungen der allgemeinen Formel IV mit Z = Wasserstoff und der reaktiven Derivate VII mit Verbindungen der allgemeinen Formel VI erfolgt zweckmäßig unter Zusatz einer starken Base, wie z. B. Natriumethylat, Natriumhydrid oder 1.8-Diazabicyclo-(5.4.0)-undec-7-en.

Als inertes Lösungsmittel dienen z. B. Ethanol, Dimethylsulfoxid, Toluol oder Benzol. Weiterhin kommen z. B. Dimethylformamid oder Hexamethylphosphorsäuretriamid als Lösungsmittel in Betracht. Die Reaktion wird vorzugsweise bei Zimmertemperatur oder mäßig erhöhter Temperatur oder bei der Siedetemperatur des verwendeten Lösungsmittels durchgeführt. Als reaktiver Rest X kommen hier z. B. Halogenide oder Sulfonsäureestergruppen in Frage, insbesondere Chlorid, Bromid oder die p-Toluolsulfonyloxy- und die Methansulfonyloxygruppe.

Die Umsetzung der Verbindungen IV, Z = Wasserstoff, mit Aldehyden der Formel V′ nach Verfahren b2) findet unter Bedingungen statt, wie sie für die Kondensation aktivierter Methylengruppen mit Ketoverbindungen üblich sind. Vorzugsweise wird die Kondensation in Pyridin oder Dimethylformamid unter Zusatz katalytischser Mengen einer starken Base, wie z. B. Piperidin durchgeführt. Vorteilhaft setzt man dem Reaktionsgemisch ein geeignetes Lösungsmittel, wie z. B. Benzol zu, um das Reaktionswasser azeotrop abzudestillieren.

Die nachfolgende Hydrierung der entstandenen Doppelbindung wird in üblicher Weise mit katalytisch erregtem Wasserstoff bei Normaldruck oder bei erhöhtem Druck durchgeführt. Als Katalysatoren kommen Metallkatalysatoren, wie z. B. Raney-Nickel oder Palladium-Kohle, in Betracht. Als Lösungmittel sind z. B. Essigsäure oder niedere Alkohole, im Falle von Carbonsäuren IV auch wässriges Alkali geeignet.

Unter der in eine Carboxylfunktion überführbaren Gruppe W soll insbesondere die Nitrilgruppe oder ein Rest verstanden werden, der sich oxidativ in die Carboxylfunktion überführen läßt. Als oxidierbare Gruppen kommen vorzugsweise die Hydroxymethyl-, die Aminomethyl- und die Formylgruppe oder funktionelle Derivate dieser Gruppen in Frage. Die Oxidation läßt sich mit den üblichen Oxidationsmitteln, wie z. B. Mangan-IV-Verbindungen, Permanganaten, Dichromaten, im Falle der Formylgruppe auch mit Luftsauerstoff und Silberoxid, durchführen.

Die gegebenenfalls im Anschluß an die Kondensation zu den Verbindungen der allgemeinen Formel IV durchzuführende Umwandlung des Substituenten W erfolgt beispielsweise durch Verseifen der Carbonsäureester zu den entsprechenden Carbonsäuren mit Mineralsäuren oder Alkalihydroxiden in einem polaren Lösungsmittel (wie Wasser, Methanol, Ethanol, Dioxan oder Aceton). Vorteilhaft wird die Verseifung mit einer starken Base (wie Natrium- oder Kaliumhydroxid) in einem Gemisch aus Methanol und Wasser bei Raumtemperatur oder bei mäßig erhöhten Temperaturen durchgeführt. Umgekehrt kann man aber auch die Carbonsäuren in üblicher Weise verestern oder Ester mit einem bestimmten Rest $R_3$ durch Umestern in einen Ester mit einem anderen Rest $R_3$ umwandeln. Die Veresterung der Carbonsäuren wird zweckmäßig in Gegenwart eines sauren Katalysators, wie z. B. Chlorwasserstoff, Schwefelsäure, p-Toluolsulfonsäure, oder eines stark sauren Ionenaustauschharzes vorgenommen.

Umesterungen hingegen erfordern den Zusatz einer geringen Menge einer basischen Subtanz, z. B. eines Alkali- oder Erdalkalihydroxids oder eines Alkalialkoholats. Für die Veresterung der Carboxylgruppe bzw. für eine Umesterung eignen sich prinzipiell alle Alkohole. Bevorzugt sind die niederen einwertigen Alkohole, wie Methanol, Ethanol oder Propanol, sowie mehrwertige Alkohole, z. B. Glycerin oder Alkohole mit anderen funktionellen Gruppen, wie z. B. Ethanolamin.

Die erfindungsgemäßen, von den Carbonsäuren der allgemeinen Formel I abgeleiteten Amide werden bevorzugt nach an sich bekannten Methoden aus den Carbonsäuren oder ihren reaktiven Derivaten (wie z.

B. Carbonsäurehalogeniden, -estern, -aziden, -anhydriden oder gemischten Anhydriden) durch Umsetzung mit Aminen hergestellt. Als Aminkomponenten kommen z. B. Ammoniak, Alkylamine, Dialkylamine, aber auch Aminoalkohole, wie z. B. Ethanolamin und 2-Aminopropanol, sowie Aminosäuren, wie z. B. p-Aminobenzoesäure, β-Alanin und andere in Frage. Andere wertvolle Aminkomponenten sind Alkyl-, Aralkyl- und Arylpiperazine.

Die Herstellung der obigen Amide kann aber auch durch partielle Verseifung der von den erfindungsgemäßen Carbonsäuren abgeleiteten Nitrile erfolgen. Die Verseifung erfolgt in verdünnten Mineralsäuren bei mäßig erhöhten Temperaturen, in alkalischer Hydroperoxidlösung oder vorteilhaft in 98proz. Schwefelsäure oder Polyphosphorsäure.

Zur Herstellung von Salzen mit pharmakologisch verträglichen organischen oder anorganischen Basen, wie z. B. Natriumhydroxid, Kaliumhydroxid, Calciumhydroxid, Ammoniumhydroxid, Methylglukamin, Morpholin oder Ethanolamin, können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsmittel in Wasser oder Öl, wie z. B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungmittel, Lösungsvermittler und/oder Puffer enthält. Derartige Zusätze sind z. B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z. B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gegebenenfalls gleichzeitige durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg/Tag in einer oder mehreren Anwendungen pro Tag wirksam, um die gewünschten Resultate zu erhalten.

Bevorzugt im Sinne der Erfindung sind außer den in den Beispielen genannten Verbindungen der Formel I sowie deren Estern und Amiden die folgenden:
7-Phenyl-2-(2-thienylsulfonyl)heptansäure
7-(4-Methylphenyl)-2-(2-thienylsulfonyl)heptansäure
7-(4-Methoxyphenyl)-2-(2-thienylsulfonyl)heptansäure
7-(4-Chlorphenoxy)-2-(2-thienylsulfonyl)heptansäure
4-[2-(4-Chlorphenyl)ethoxy]-2-(2-thienylsulfonyl)buttersäure
5-(4-Chlorphenyl)-2-(2-thienylsulfonyl)-4-pentinsäure
8-(4-Chlorphenyl)-2-(2-thienylsulfonyl)octansäure
7-(4-Chlorphenyl)-2-(2-thienylthio)heptansäure
7-(4-Chlorphenyl)-2-(2-thienyloxy)heptansäure
7-(4-Chlorphenylthio)-2-(2-thienyloxy)heptansäure
7-(4-Chlorphenylamino)-2-(2-thienylsulfonyl)heptansäure
7-(4-Chlorphenyl)-2-(2-methyl-3-furanylthio)heptansäure
7-(4-Chlorphenyl)-2-(1-methyl-2-imidazolyl)heptansäure
7-(4-Chlorphenyl)-2-(2-pyridylthio)heptansäure
7-(4-Chlorphenyl)-2-(4-pyridylthio)heptansäure
7-(4-Chlorphenyl)-2-(3-pyridylsulfonyl)heptansäure
8-(2-Thienyl)-2-(4-methylphenoxy)octansäure
8-(2-Thienyl)-2-(4-methylphenylthio)octansäure
8-(2-Thienyl)-2-(phenylsulfonyl)octansäure
8-(2-Thienyl)-2-(4-tert.-butylphenylsulfonyl)octansäure
8-(2-Thienyl)-2-(4-fluorphenylsulfonyl)octansäure
8-(2-Thienyl)-2-(4-chlorphenylsulfonyl)octansäure

8-(2-Thienyl)-2-(3-trifluormethylphenylsulfonyl)octansäure
8-(2-Thienyl)-2-(4-methoxyphenylsulfonyl)octansäure
8-(2-Thienyl)-2-(2-phenylethylsulfonyl)octansäure
8-(2-Thienyl)-2-(4-methylphenylsulfinyl)octansäure
8-(2-Furanyl)-2-(4-methylphenylsulfonyl)octansäure
8-(2-Thienyl)-2-(2-phenylethenylsulfonyl)octansäure
8-(2-Thienyl)-2-(4-chlorcinnamylsulfonyl)octansäure
8-(3-Thienyl)-2-(4-methylphenylsulfonyl)octansäure
8-(5-Methyl-2-thienyl)-2-(4-methylphenylsulfonyl)octansäure
8-(5-Chlor-2-thienyl)-2-(4-methylphenylsulfonyl)octansäure
8-(3-Methoxy-2-thienyl)-2-(4-methylphenylsulfonyl)octansäure
8-(2-Thienyl)-2-2-(4-methylphenylsulfinyl)octansäure
8-(4-Pyridyl)-2-(4-methylphenylsulfonyl)octansäure
8-(3-Pyridyl)-2-(4-methylphenylsulfonyl)octansäure
8-(2-Pyridyl)-2-(4-methylphenylsulfonyl)octansäure
8-(4-Ethyl-2-pyridyl)-2-(4-methylphenylsulfonyl)octansäure
7-(3-Methyl-2-pyridyl)-8-hydroxy-2-(4-methylphenylsulfonyl)heptansäure
7-(3,5-Dimethyl-4-oxazolyl)-2-(4-methylphenylsulfonyl)heptansäure

**Beispiel 1**

2-(4-Methylphenylsulfonyl)-8-(2-thienyl)octansäure

Man versetzt eine Lösung von 28 mmol Natriumethylat (aus 0,644 g Natrium) in 70 ml abs. Ethanol unter Rühren mit einer Lösung von 6,78 g (28 mmol) 4-Methylphenylsulfonylessigsäureethylester in 150 ml abs. Ethanol und erhitzt 1 Stunde auf Rückflußtemperatur. Dann tropft man 7,0 g (28 mmol) 6-(2-Thienyl)-hexylbromid in 20 ml abs. Ethanol zu und erhitzt erneut 6 Stunden unter Rückfluß. Anschließend engt man ein, nimmt in Wasser auf und extrahiert das Gemisch mit Ether. Die Etherextrakte werden getrocknet und eingedampft. Den Rückstand chromatographiert man mit einem Gemisch von Heptan und 2-Butanon (2 : 1) an Kieselgel. Man erhält 8,4 g (73 % d. Th.) 2-(4-Methylphenylsulfonyl)-8-(2-thienyl)octansäureethylester, farbloses Öl.

3,3 g (8 mmol) dieses Esters werden in einem Gemisch von 8,5 ml 1 N Kalilauge und 50 ml Methanol 6 Stunden bei Raumtemperatur gerührt. Dann destilliert man das Methanol ab, verdünnt den Rückstand mit Wasser und wäscht mit Ether. Die wässrige Phase wird mit Kohle geklärt, angesäuert und das abgeschiedene Öl in Ether extrahiert. Die Etherextrakte werden getrocknet und eingedampft. Man erhält als Rückstand 3,0 g farbloses Öl. Das Öl wird mit einer Lösung von 0,65 g (7,8 mmol) Natriumhydrogencarbonat in 20 ml Wasser gerührt, bis eine klare Lösung entstanden ist. Dann dampft man ein. Man erhält als Rückstand 2,6 g (81 % d. Th.) Natrium-2-(4-Methylphenylsulfonyl)-8-(2-thienyl)octanoat, Fp. 129 bis 131 °C.

Das als Ausgangsmaterial verwendete 6-(2-Thienyl)hexylbromid kann aus 6-Oxo-6-(2-thienyl)hexansäure (Papa und Schwenk, J. Am. Chem. Soc. 69, 3022) auf folgende Weise hergestellt werden:
Ein Gemisch von 30,0 g (0,14 mol) 6-Oxo-6-(2-thienyl)hexansäure (Fp. 77 °C), 26,3 g (0,47 mol) Ätzkali, 17,5 ml (0,35 mol) Hydrazinhydrat, 4 ml Wasser und 180 ml Diethylenglykol werden 2 Stunden auf Rückflußtemperatur erhitzt. Dann destilliert man am absteigenden Kühler das Diethylglykol ab, nimmt den Rückstand in Wasser auf und wäscht die Neutralanteile mit Ether aus. Die wässrige Phase wird anschließend angesäuert und mit Ether extrahiert. Die Etherextrakte werden getrocknet und eingedampft. Den Rückstand destilliert man im Vakuum. Man erhält 16,1 g (58 % d. Th.) 6-(2-Thienyl)hexansäure, Sdp. 135 bis 137 °/0,4 mbar.

Man versetzt unter Rühren bei 0 °C eine Suspension von 7,6 g (0,2 mol) Lithiumaluminiumhydrid in 200 ml abs. Ether mit einer Lösung von 16 g (80 mmol) dieser Carbonsäure in 100 ml abs. Ether und rührt das Gemisch noch 2 Stunden ohne Kühlung, dann 1 Stunde unter Rückfluß nach. Anschließend zersetzt man mit Wasser und dampft die organische Phase ein. Man erhält als Rückstand 14,7 g (98 % d. Th.) 6-(2-Thienyl)hexanol, farbloses Öl.

20 g (0,108 mol) dieses Hexanols werden unter Eiskühlung vorgelegt und tropfenweise mit 29,3 g (0,108 mol) Phosphortribromid versetzt. Man rührt noch 1 Stunde unter Eiskühlung, dann 4 Stunden bei 100 °C nach und gießt auf Eis. Das Gemisch extrahiert man mit Ether und dampft die Extrakte ein. Den Rückstand destilliert man im Vakuum. Man erhält 15,3 g (57 % d. Th.) 6-(2-Thienyl)hexylbromid, Sdp. 115 bis 117 °C/0,2 mbar.

**Beispiel 2**

7-(4-Chlorphenyl)-2-(2-thienyl)sulfonylheptansäure

Zu einer Lösung von 40 mmol Natriumethylat (aus 0,92 g Na) in 75 ml abs. Ethanol tropft man nacheinander unter Rühren 9,37 g (40 mmol) (2-Thienyl)sulfonylessigsäureethylester in 35 ml Ethanol und 10,46 g (40 mmol) 5-(4-Chlorphenyl)pentylbromid in 30 ml Ethanol.

Anschließend erhitzt man das Reaktionsgemisch 6 Stunden auf Rückflußtemperatur, dampft ein und nimmt den Rückstand in Ether auf. Man wäscht die Etherlösung mit Wasser, trocknet und engt sie ein. Der Rückstand wird mit Methylenchlorid an Kieselgel chromatographiert. Man erhält 8,6 g (52 % d. Th.) 7-(4-Chlorphenyl)-2-(2-thienyl)sulfonylheptansäureethylester, Fp. 64 bis 66 °C.

7,3 g (17,5 mmol) dieses Esters werden in einem Gemisch von 40 ml 1 N Kalilauge und 120 ml Methanol 2 Stunden bei 40 °C gerührt. Dann dampft man das Methanol ab, verdünnt den Rückstand mit Wasser und wäscht mit Ether. Anschließend wird die wässrige Lösung mit Kohle geklärt und angesäuert. Der abgeschiedene Niederschlag wird in Ether aufgenommen. Man trocknet und dampft ein. Ausbeute: 6,5 g (96 % d. Th.) 7-(4-Chlorphenyl)-2-(2-thienylsulfonyl)heptansäure, Fp. 106 bis 108 °C.

Der als Ausgangsmaterial verwendete (2-Thienyl)sulfonylessigsäureethylester kann aus Lithium-(2-thienyl)sulfinat und Bromessigester als farbloses Öl erhalten werden. Ausbeute: 78 % d. Th., Sdp. 156 bis 157 °C/0,2 mbar.

**Patentansprüche**

**1.** Carbonsäurederivate der Formel I

$$\begin{array}{c} R_1\text{-A-CH-COOH} \\ \quad\;| \\ \quad\;\;\text{Y-B-}R_2 \end{array} \qquad (I)$$

in welcher

| | |
|---|---|
| $R_1$ und $R_2$ | unabhängig voneinander einen gegebenenfalls durch Fluor, Chlor, Brom, $C_1$-$C_6$ Alkyl, $C_1$-$C_6$ Alkoxy, Trifluormethyl oder Cyano substituierten $c_6$-$c_{14}$ Arylrest oder einen gegebenenfalls durch Fluor, Chlor, Brom, $C_1$-$C_6$ Alkyl oder $C_1$-$C_6$ Alkoxy substituierten Thiophen-, Pyridin- oder Furanrest, |
| A | einen geradkettigen oder verzweigten, gesättigten oder ungesättigten zweiwertigen aliphatischen Kohlenwasserstoffrest mit 1 bis 10 Kohlenstoffatomen, der ein Sauerstoff-, Schwefel- oder Stickstoffatom als Kettenglied enthalten kann und gegebenenfalls durch einen Hydroxylrest substituiert ist, |
| Y | die Gruppe $S(O)_n$- oder O, |
| n | die Zahlen 0, 1 oder 2 und |
| B | einen Valenzstrich oder einen gesättigten oder ungesättigten zweiwertigen aliphatischen Kohlenwasserstoffrest mit 1 bis 5 Kohlenstoffatomen |

bedeuten,

sowie deren physiologisch unbedenkliche Salze, Ester und Amide, mit der Maßgabe, daß

a) $R_1$ und $R_2$ nicht gleichzeitig einen Arylrest bedeuten und

b) im Falle eines ungesättigten Kohlenwasserstoffrestes A, der ein Heteroatom als Kettenglied

enthält, das Heteroatom nicht an einem ungesättigten aliphatischen Kohlenstoffatom gebunden ist.

**2.** Verfahren zur Herstellung von Carbonsäurederivaten der Formel I

$$\begin{array}{l} R_1\text{-A-CH-COOH} \\ \qquad\quad | \\ \qquad\ \text{Y-B-}R_2 \end{array} \qquad (I)$$

in welcher

- $R_1$ und $R_2$ unabhängig voneinander einen gegebenenfalls durch Fluor, Chlor, Brom, $C_1$-$C_6$ Alkyl $C_1$-$C_6$ Alkoxy, Trifluormethyl oder Cyano substituierten $C_6$-$C_{14}$ Arylrest oder einen gegebenenfalls durch Fluor, Chlor, Brom, $C_1$-$C_6$ Alkyl oder $C_1$-$C_6$ Alkoxy substituierten Thiophen-, Pyridin- oder Furanrest,
- A einen geradkettigen oder verzweigten, gesättigten oder ungesättigten, zweiwertigen aliphatischen Kohlenwasserstoffrest mit 1 bis 10 Kohlenstoffatomen, der ein Sauerstoff-, Schwefel- oder Stickstoffatom als Kettenglied enthalten kann und gegebenenfalls durch einen Hydroxylrest substituiert ist,
- Y die Gruppe $S(O)_n$ oder O,
- n die Zahlen 0, 1 oder 2 und
- B einen Valenzstrich oder einen gesättigten oder ungesättigten zweiwertigen aliphatischen Kohlenwasserstoffrest mit 1 bis 5 Kohlenstoffatomen

bedeuten,

sowie von deren physiologisch unbedenklichen Salzen, Estern und Amiden, mit der Maßgabe, daß

a) $R_1$ und $R_2$ nicht gleichzeitig einen Arylrest bedeuten und

b) im Falle eines ungesättigten Kohlenwasserstoffrestes A, der ein Heteroatom als Kettenglied enthält, das Heteroatom nicht an einem ungesättigten aliphatischen Kohlenstoffatom gebunden ist.

dadurch gekennzeichnet, daß man

A) eine Gruppe der allgemeinen Formel I

$$\begin{array}{l} \text{Z-CH-W} \\ \quad\ \ | \\ \quad\ \ \text{X} \end{array} \qquad (II),$$

in welcher

- W -$COOR_3$ oder eine andere in die Carboxylfunktion überführbare Gruppe,
- $R_3$ eine $C_1$-$C_6$ Alkylgruppe,
- X hier und in allen folgenden Erläuterungen eine reaktive Gruppe und

Z Wasserstoff oder die Gruppe $R_1$-A-, in welcher $R_1$ und A die oben angegebene Bedeutung haben,

darstellt,

in an sich bekannter Weise mit einer Gruppe der allgemeinen Formel III

$R_2$-B-YH (III),

in welcher $R_2$, B und Y die oben angegebene Bedeutung haben, zu einer Verbindung der allgemeinen Formel IV

```
Z-CH-W
  |
  Y-B-R2          (IV),
```

umsetzt und im Anschluß daran gegebenenfalls

a) für den Fall, daß Y ein Schwefelatom darstellt, zur Gruppe Y = SO oder $SO_2$ oxidiert oder

b) für den Fall, daß Z Wasserstoff bedeutet, entweder

$b_1$) mit einer Verbindung der allgemeinen Formel V

$R_1$-A-X (V),

alkyliert oder

$b_2$) mit einer Verbindung der allgemeinen Formel V'

$R_1$-A'-CHO (V'),

in welcher A' einen um die $CH_2$-Gruppe verkürzten Kohlenwasserstoffrest A nach oben genannter Definition bedeutet, kondensiert und im Anschluß an die Kondensation die entstandene Doppelbindung hydriert,

oder dass man

B) eine Verbindung der allgemeinen Formel VI

```
R1-A-CH-W
     |
     YH           (VI)
```

mit einer Gruppe der allgemeinen Formel VII

$R_2$-B-X (VII)

zu einer Verbindung der allgemeinen Formel IV umsetzt und an den erhaltenen Verbindungen der allgemeinen Formel IV im Anschluß an die vorgenannten Verfahrensschritte die Gruppe W in eine freie Carboxylfunktion oder deren unbedenkliche Salze, einen physiologisch verträglichen Ester oder ein Amid umwandelt.

3. Arzneimittel, enthaltend eine Verbindung gemäß Anspruch 1 neben üblichen Träger- und Hilfsstoffen.

4. Verwendung von Verbindungen gemäß Anspruch 1 zur Herstellung von Arzneimitteln zur Behandlung von Stoffwechselerkrankungen.

## Claims

1. Carboxylic acid derivatives of the formula I

$$\begin{array}{l} R_1 - A - \underset{|}{CH} - COOH \\ \qquad\qquad Y - B - R_2 \end{array} \quad (I)$$

in which $R_1$ and $R_2$, independently of one another, signify a $C_6$-$C_{14}$-aryl radical possibly substituted by fluorine, chlorine, bromine, $C_1$-$C_6$-alkyl, $C_1$-$C_6$-alkoxy, trifluoromethyl or cyano or a thiophene, pyridine or furan radical possibly substituted by fluorine, chlorine, bromine, $C_1$-$C_6$-alkyl or $C_1$-$C_6$-alkoxy, A a straight-chained or branched, saturated or unsaturated divalent aliphatic hydrocarbon radical with 1 to 10 carbon atoms which can contain an oxygen, sulphur or nitrogen atom as chain member and is possibly substituted by a hydroxyl radical, Y the group $S(O)_n$ or 0, n the numbers 0, 1 or 2 and B a valency bond or a saturated or unsaturated divalent aliphatic hydrocarbon radical with 1 to 5 carbon atoms; as well as their physiologically acceptable salts, esters and amides, with the proviso that
a) $R_1$ and $R_2$ do not simultaneously signify an aryl radical and
b) in the case of an unsaturated hydrocarbon radical A which contains a heteroatom as chain member, the heteroatom is not connected to an unsaturated aliphatic carbon atom.

2. Process for the preparation of carboxylic acid derivatives of the formula I

$$\begin{array}{l} R_1 - A - \underset{|}{CH} - COOH \\ \qquad\qquad Y - B - R_2 \end{array} \quad (I)$$

in which $R_1$ and $R_2$, independently of one another, signify a $C_6$-$C_{14}$-aryl radical possibly substituted by fluorine, chlorine, bromine, $C_1$-$C_6$-alkyl, $C_1$-$C_6$-alkoxy, trifluoromethyl or cyano or a thiophene, pyridine or furan radical possibly substituted by fluorine, chlorine, bromine, $C_1$-$C_6$-alkyl or $C_1$-$C_6$-alkoxy, A a straight-chained or branched, saturated or unsaturated divalent aliphatic hydrocarbon radical with 1 to 10 carbon atoms which can contain an oxygen, sulphur or nitrogen atom as chain member and is possibly substituted by a hydroxyl group, Y the group $S(O)_n$ or 0, n the numbers 0, 1 or 2 and B a valency bond or a saturated or unsaturated divalent aliphatic hydrocarbon radical with 1 to 5 carbon atoms; as well as of their physiologically acceptable salts, esters and amides, with the proviso that
a) $R_1$ and $R_2$ do not simultaneously signify an aryl radical and
b) in the case of an unsaturated hydrocarbon radical A which contains a heteroatom as chain member, the heteroatom is not connected to an unsaturated aliphatic carbon atom, characterised in that one
a) reacts a group of the general formula II

$$\begin{array}{l} Z - \underset{|}{CH} - W \\ \quad\;\; X \end{array} \quad (II)$$

in which W represents $-COOR_3$ or a group convertible into the carboxyl function, $R_3$ a $C_1$-$C_6$-alkyl group, X here and in all following explanations a reactive group and Z hydrogen or the group $R_1$-A-, in which $R_1$ and A have the above-given meaning, in per se known manner with a group of the

general formula III

$R_2$ - B - YH (III),

in which $R_2$, B and Y have the above-given meaning, to give a compound of the general formula IV

$$\begin{array}{l} Z - \underset{\displaystyle Y - B - R_2}{CH} - W \end{array} \qquad (IV),$$

and possibly subsequently thereto

a) for the case that Y represents a sulphur atom, oxidises to the group Y = SO or $SO_2$ or

b) for the case that Z signifies hydrogen, either

$b_1$) alkylates with a compound of the general formula V

$R_1$ - A - X (V)

or

$b_2$) condenses with a compound of the general formula V'

$R_1$ - A' - CHO (V')

in which A' signifies a hydrocarbon radical A according to the above definition shortened by the $-CH_2-$ group, and subsequent to the condensation hydrogenates the resultant double bond or that one

B) reacts a compound of the general formula VI

$$R_1 - A - \underset{\displaystyle YH}{CH} - W \qquad (VI)$$

with a group of the general formula VII

$R_2$ - B - X (VII)

to give a compound of the general formula IV and in the compounds obtained of the general formula IV, subsequent to the above-mentioned process steps, converts the group W into a free carboxyl function or its acceptable salts, a physiologically acceptable ester or an amide.

3. Medicaments containing a compound according to claim 1, besides usual carrier and adjuvant materials.

4. Use of compounds according to claim 1 for the preparation of medicaments for the treatment of metabolic diseases.

## Revendications

1. Dérivés d'acides carboxyliques de formule I

$$\begin{array}{l} R_1-A-CH-COOH \\ \qquad\quad | \\ \qquad\quad Y-B-R_2 \end{array} \qquad (I)$$

dans laquelle

- $R_1$ et $R_2$ représentent, indépendamment, un groupe aryle en $C_6$-$C_{14}$, éventuellement substitué par un atome de fluor, de chlore, de brome, par un groupe alkyle en $C_1$-$C_6$, alcoxy en $C_1$-$C_6$, trifluorométhyle ou cyano, ou un groupe thiophène, pyridine ou furane éventuellement substitué par un atome de fluor, de chlore, de brome, par un groupe alkyle en $C_1$-$C_6$ ou alcoxy en $C_1$-$C_6$,
- A représente un groupe hydrocarboné aliphatique bivalent, saturé ou insaturé, linéaire ou ramifié, comportant 1 à 10 atomes de carbone, qui peut contenir, comme chaînon, un atome d'oxygène, de soufre ou d'azote, et qui est éventuellement substitué par un groupe hydroxyle,
- Y représente le groupe $S(O)_n$ ou O,
- n vaut 0, 1 ou 2 et
- B représente une valence libre ou un groupe hydrocarboné aliphatique bivalent, saturé ou insaturé, comportant 1 à 5 atomes de carbone,

ainsi que leurs sels, esters et amides physiologiquement acceptables, étant entendu que

a) $R_1$ et $R_2$ ne représentent pas simultanément un groupe aryle, et

b) dans le cas d'un groupe hydrocarboné A insaturé, qui contient un hétéroatome comme chaînon, l'hétéroatome n'est pas lié à un atome de carbone aliphatique insaturé.

**2.** Procédé de préparation de dérivés d'acides carboxyliques de formule I

$$\begin{array}{l} R_1-A-CH-COOH \\ \qquad\quad | \\ \qquad\quad Y-B-R_2 \end{array} \qquad (I)$$

dans laquelle

- $R_1$ et $R_2$ représentent, indépendamment, un groupe aryle en $C_6$-$C_{14}$, éventuellement substitué par un atome de fluor, de chlore, de brome, par un groupe alkyle en $C_1$-$C_6$, alcoxy en $C_1$-$C_6$, trifluorométhyle ou cyano, ou un groupe thiophène, pyridine ou furane éventuellement substitué par un atome de fluor, de chlore, de brome, par un groupe alkyle en $C_1$-$C_6$ ou alcoxy en $C_1$-$C_6$,
- A représente un groupe hydrocarboné aliphatique bivalent, saturé ou insaturé, linéaire ou ramifié, comportant 1 à 10 atomes de carbone, qui peut contenir, comme chaînon, un atome d'oxygène, de soufre ou d'azote, et qui est éventuellement substitué par un groupe hydroxyle,
- Y représente le groupe $S(O)_n$ ou O,
- n vaut 0, 1 ou 2 et
- B représente une valence libre ou un groupe hydrocarboné aliphatique bivalent, saturé ou insaturé, comportant 1 à 5 atomes de carbone,

ainsi que leurs sels, esters et amides physiologiquement acceptables, étant entendu que

a) $R_1$ et $R_2$ ne représentent pas simultanément un groupe aryle, et

b) dans le cas d'un groupe hydrocarboné A insaturé, qui contient un hétéroatome comme chaînon, l'hétéroatome n'est pas lié à un atome de carbone aliphatique insaturé,

caractérisé en ce que

soit

A) on fait réagir un groupe de formule générale I

Z-CH-W (II),
|
X

dans laquelle

- W représente un groupe $-COOR_3$, ou un autre groupe transformable en un groupe fonctionnel carboxyle,
- $R_3$ représente un groupe alkyle en $C_1$-$C_6$,
- X représente, ici et dans toutes les significations suivantes, un groupe réactif, et
- Z représente l'hydrogène ou le groupe $R_1$-A-, dans lequel $R_1$ et A ont les significations mentionnées ci-dessus,

de manière connue en soi avec un groupe de formule générale III

$R_2$-B-YH (III),

dans laquelle $R_2$, B et Y ont les significations mentionnées ci-dessus, en un composé de formule générale IV

Z-CH-W
|
Y-B-$R_2$ (IV),

et ensuite éventuellement

a) dans le cas où Y représente un atome de soufre, on oxyde en groupe Y = SO ou $SO_2$, ou

b) dans le cas où Z représente l'hydrogène, soit

b₁) on alkyle avec un composé de formule générale V

$R_1$-A-X (V),

soit

$b_2$) on condense avec un composé de formule générale V'

$R_1$-A'-CHO (V'),

dans laquelle A' représente un groupe hydrocarboné A selon la définition ci-dessus, raccourci d'un motif $CH_2$, et après la condensation, on hydrogène la double liaison formée,

soit

B) on fait réagir un composé de formule générale VI

$R_1$-A-CH-W
|
YH (VI)

avec un groupe de formule générale VII

$R_2$-B-X (VII),

en un composé de formule générale IV et sur le composé de formule générale IV obtenu, à la suite de l'étape de procédé mentionnée précédemment, on transforme le groupe W en une fonction

carboxyle libre ou en son sel, ester ou amide physiologiquement acceptable.

3. Médicaments contenant un composé selon la revendication 1, en plus de supports et adjuvants usuels.

4. Utilisation des composés selon la revendication 1, pour la préparation de médicaments destinés au traitement de troubles du métabolisme.